# EUROPEAN PATENT SPECIFICATION

(11) **EP 2 384 726 B1**
(45) Date of publication and mention of the grant of the patent: **13.02.2013**
(21) Application number: 10382112.0
(22) Date of filing: 06.05.2010
(51) Int. Cl.: A61F 5/37

(54) **Fastening abdominal belt for fixing the patients on the bed**
Befestigungsgurt für den Bauch zur Befestigung des Patienten am Bett
Ceinture abdominale de sécurité de fixation du patient au lit

(43) Date of publication of application: 09.11.2011
(73) Proprietor: Medi Care System, S.L., 08302 Mataró (ES)
(72) Inventor: Montero Alfonso, Manuel, 08302 Mataró (ES)
(74) Representative: Manresa Val, Manuel

(56) References cited:
- WO-A1-2007/144134
- DE-U1-202006 009 398

## Description

Abdominal fastening belt for securing patients to a bed, of the type comprising a first strap joined to a fastening band that is secured to the bed, having a first section with eyelets, arranged successively and separated a certain distance from each other, and a second band, joined to an abdominal belt that has two ends which when joined together, wrap around and protect the patient's body, with the fastening band and the abdominal belt also being joined together, characterized in that the first strap has a second section of eyelets, double and unfolded, with at least three eyelets near the fastening belt, with the distance between the first and second eyelet different from the distance between the second and third eyelet.

The document DE 20 2006 009 398 U1 is regarded as the closest prior art.

### BACKGROUND TO THE INVENTION

So, different registers are known in the state of the art relating to fastening belt for patients in beds and chairs.

In fact, the actual inventor is also the inventor of Spanish Utility Model No. 9900296 "DEVICE FOR SECURING A HANDICAPPED PERSON TO A CHAIR", 1999, which relates to a device for fastening a handicapped person to a chair, particularly a chair with a backrest, such as a wheelchair or a chair for handicapped people, which device is of the type that has means for fastening some part of the handicapped person's body to said backrest, characterized in that it has a belt piece the ends of which are provided with means for being fastened together, in an adjustable, extendible manner, behind said backrest, while winding around the handicapped person's abdomen and the backrest, which belt piece has, extending from the central part thereof, either a pectoral section for securing the thorax of said person above the shoulders to the backrest, or a perineal part for securing the person's pelvis to the backrest, or a combination of both belt pieces, pectoral and perineal, which have, at the respective free ends thereof, means for being fastened together, in an adjustable and extendible fashion, with said belt piece at the rear of the backrest.

### BRIEF DESCRIPTION OF THE INVENTION

This invention is an improvement in the sector of fastening systems for patients in beds.

These fastening systems have become necessary due to the fact that certain patients require being immobilised in bed because they are disorientated, aggressive or disordered, thereby avoiding the risk of injuring themselves or the possibility of injuring their carers, as well as avoiding possible falls from the bed or stretcher.

These devices do not interfere with changing the patient's posture to prevent bedsores. The closing system in the invention also guarantees avoiding the risk of uncontrolled opening through handling by the same patient or non-authorised personnel.

The inventor has decided to go one step further. One of the problems affecting these fastening systems is that they must be adapted to different size beds and mattresses in hospitals.

Thus, the inventor, in the fastening belt to bed, has arranged in the first section nearest the wide band at the base, a series of eyelets strategically separated from each other by certain distances. One of the improvements consists in including a series of eyelets arranged successively, at different distances between one and the next, so that by superimposing two eyelets all the necessary distances for adjusting the belt can be achieved.

In fact, said predetermined distance between the eyelets is usually proportional, and this will be described in detail in the particular embodiment.

This is because when the belt base is fitted to the bed, using the strap, the latter is tensed, and one of the eyelets at the end of the band is made to line up with one of the eyelets in the second section of the strap.

The fact that in this second section the distance between the eyelets is different means that when adjusting the belt there are greater possibilities of finding a pair of aligned eyelets, since different eyelet combinations are produced, which also means that there are many possibilities of adapting and adjusting the strap.

Another advantage is that in the abdominal belt additional fastening means have been arranged, for example, of the hook/loop type, which means that when the abdominal belt is closed, it is better positioned while also securing the two ends of the belt band, and preventing the belt from moving. This manoeuvre makes it easier for the carer to put the belt on the patient, and avoids some patients trying to make a hole between the ends of the abdominal belt bands to try and break loose.

The abdominal belt, which wraps around the patient's body, also includes identification means, so that the nursing staff, even when the fastening system is changed, can quickly adjust said abdominal belt on the patient, without having to test whether the adjustment is correct.

These identification or eyelet coding means that are used, can also be used to detect any possible abnormalities in the patient, as they provide the health care staff with information on whether or not it is necessary to adjust the belt more (indication that a patient has possibly lost weight) or, on the contrary, if the patient requires a wider belt diameter, which would indicate that the patient is gaining weight (retaining liquids, internal effusions, etc.).

An object of this invention is an abdominal fastening belt for securing patients to a bed, of the type comprising a first strap, joined to a fastening band that is attached to the bed, with a first section of eyelets, arranged successively, and separated a certain distance from each other, and a second strap, joined to an abdominal belt that has two ends which when joined together, wrap around and protect the patient's body, with the fastening band and the abdominal belt also being joined together, characterized in that the first strap has a second section of eyelets, double and unfolded, with at least three eyelets near the fastening band, with the distance between the first and second eyelet being different from the distance between the second and third eyelet.

### BRIEF DESCRIPTION OF THE DRAWINGS

In order to facilitate the explanation, four sheets of drawings are attached to this specification, which represent a practical embodiment that is provided as a non-limiting example of the scope of this invention:
- Figure 1 is the object of this invention, showing the two wide bands that make up the base of the belt and the actual belt itself.
- Figure 2 shows the detail of II in Figure 1.
- Figure 3 shows the detail of the abdominal belt.
- Figure 4 is a perspective view from below, of the belt open.
- Figures 5a and 5b are examples of the several eyelet alignment combinations.

### CONCRETE EMBODIMENT OF THIS INVENTION

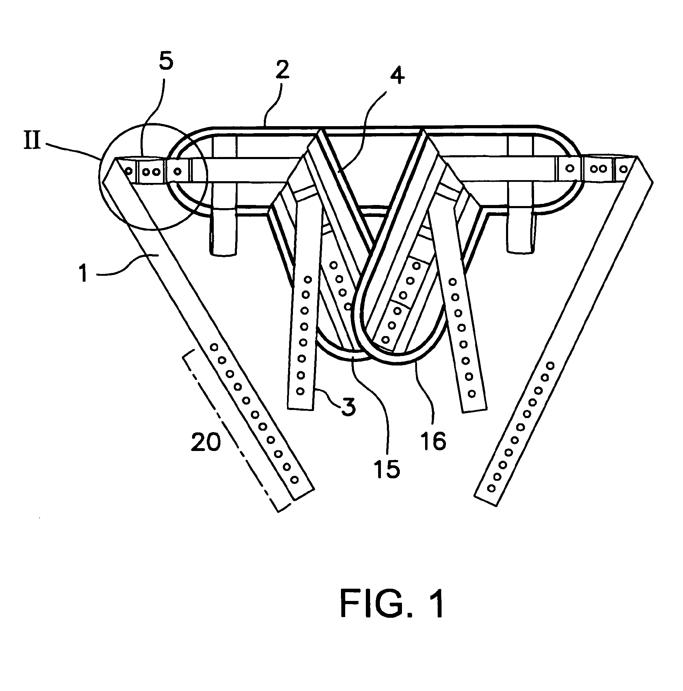

So Figure 1 shows a fastening band 2 with a first strap 1, a first section 20 and a second section 5, an abdominal belt 4 with its ends 15, 16 and a second strap 3.

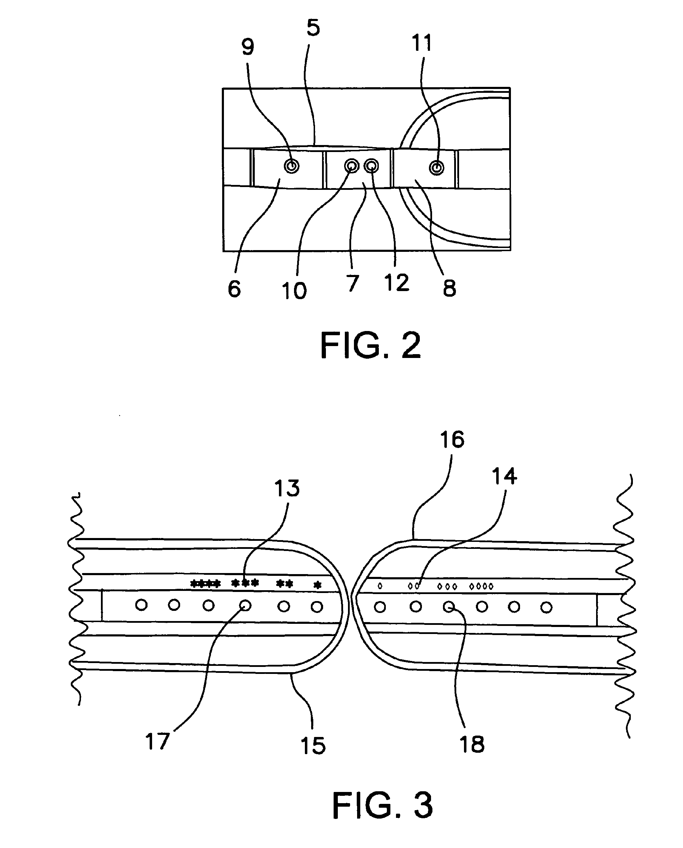

Figure 2 shows second section 5, a first segment 6 with a first eyelet 9, a second segment 7 with a second eyelet 10 and a third eyelet 12 and a third segment 8 with a fourth eyelet 11.
Figure 3 shows ends 15, 16 identification means 13, 14 and eyelets 17, 18.

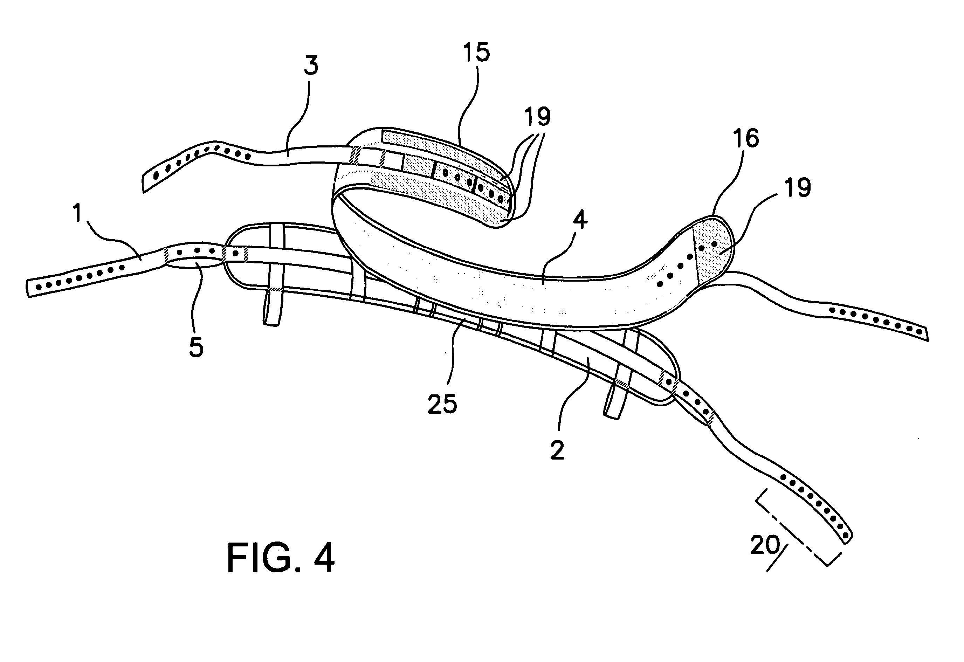

Figure 4 illustrates fastening band 2 with the first strap 1, first section 20 and second section 5, abdominal belt 4 with its internal attachment means 19 located on ends 15, 16 and second strap 3.

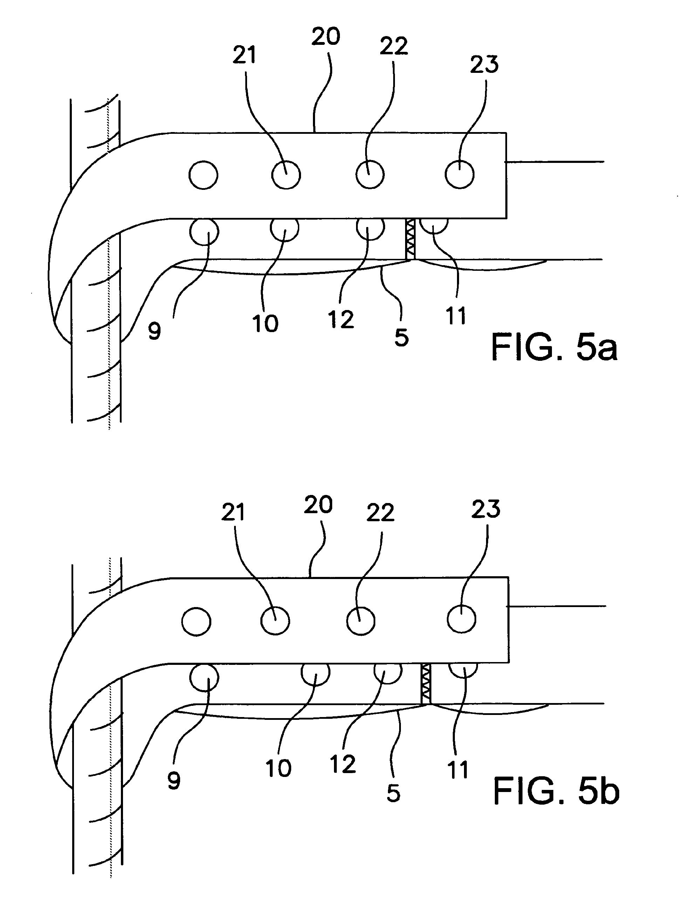

Lastly, Figures 5a and 5b represent the second section 5, with the first eyelet 9, the second eyelet 10, the third eyelet 12 and the fourth eyelet 11, and the first section 20 with a fifth eyelet 21, a sixth eyelet 22 and a seventh eyelet 23.

Second strap 3 integral with belt 4 is the one that is used to limit the patient's movements when changing their posture.

The division into segments 6, 7 and 8 corresponds to the need to separate them and sew the second section 5 in the rear part thereof, so as to prevent the pivots in the mechanical closing system falling down once assembled.

Fastening band 2 and abdominal belt 4 are joined together by a hinge 25 that allows for the patient's posture to be changed.

In a concrete embodiment, when the medical professional, for example a nurse, wants to fasten a patient to the bed, he would proceed as follows with the belt that is the object of this invention.

First of all, the fastening band 2 is arranged on the bed, on top of it, leaving first straps 1 that are tightly attached to the fastening band 2, hanging down.

First straps 1 are attached to the bed by passing first strap 1 through a cross member in the bed that is integral with the mattress (that is moved with said mattress in articulated beds). As shown in Figures 5a and 5b, making an eyelet on first section 20 of first strap 1, coincide with an eyelet on second section 5 of first strap 1, and joining them by means of a mechanical pivot and a safety button, for example, like the ones distributed by the applicant under the trade name SALVAFIX.

To secure the first straps 1 to the bed, proceed as follows:
On first strap 1, which corresponds to fastening band 2, a first section 20 has been arranged, where a series of eyelets is provided, separated a certain distance from each other.

Then there is a second section 5, double, unfolded, with at least three eyelets (see detail in Fig. 2), although they could be more, that are placed near to or even on top of fastening band 2.

The distance, within the first segment 6, between the first eyelet 9 and the second eyelet 10 is different from the distance between the second eyelet 10 and the fourth eyelet 11.

Said certain distance is usually proportional to the distance between the first 9 and third eyelet 12, and in this particular embodiment it would be approximately a quarter of the distance in this second section 5, or half the distance between the eyelets of the first section 20.

When fastening belt 2 is locked on the bed, thanks to first strap 1, this latter is tensed, and one of the eyelets 21, 22 or 23 in the first section is made to align with one of the eyelets 9, 10 or 12 in the second section (Fig. 5a).

The fact that in the second section 5, the distance between the second eyelet 10 and the third eyelet 12 is different from the distance that separates the first eyelet 9 and the third eyelet 12, means that when adjusting, there is a greater possibility of finding a pair of aligned eyelets, because different eyelet combinations are formed, which leads to many possibilities of adapting first strap 1 and adjusting fastening band 2 to the bed, for different bed and mattress dimensions. This point is very important as the patient is perfectly fastened when fastening band 2 is firmly secured to the bed.

As can be seen in Figure 5a, if the adjustment is as shown, eyelet 10 could be aligned with eyelet 22 and eyelet 9 with eyelet 21. However, if there were any slight play, it could be adjusted a little more by fourth eyelet 11 which would be made to coincide with eyelet 23 (Fig. 5b), so that this belt can be adjusted perfectly to any type of bed.

Said fourth eyelet 11 is not basic, although in the event that second section 5 were to include a fourth eyelet 11, placed within said fastening band 2, it could be used in narrower beds or stretchers.

This embodiment in Figs. 1 and 2 is very useful in those medical centres or hospitals that have a wide variety of beds, at different heights, with different size mattresses, and which therefore require greater versatility when adjusting the belt to the bed.

As shown in Figure 4, on abdominal belt 4 internal attachment means 19 have been arranged, which secure ends 15, 16 of abdominal belt 4 around the perimeter.

Said securing means can be of the VELCRO hook/loop type. It is intended that abdominal belt 4 does not move and that there is no play. Patients, by moving, can end to create play on abdominal belt 4 and therefore the patient can break free.

This way, when using securing means 19, to both in end 15 as in the other end 16, is avoided certain play on abdominal belt 4 and it is firmly secured, in a non-movable way.

If also the patient is an habitual patient, the nurse will have the medical identification 13, 14 (Figure 3), concerning the eyelets 17, 18, from ends 15, 16 noted down and will be able to apply it quickly.

These identification means are important because sometimes carers, due to the patient's psychic condition, may not have much time to keep trying different combinations until they find the right one.

At the same time, these identification means can be used to know whether the patient has lost weight, or has some other condition, as identification means 13, 14 would vary; in other words, the combination would not be the same. In fact, each eyelet is unequivocally identified by an element that is different to the ones used for other eyelets.

This invention describes a new abdominal fastening belt for securing patients to a bed. The examples mentioned herein are non-limiting in terms of the invention, and therefore it could have different applications and/or adaptations, all within the scope of the following claims.

## Claims

1. Abdominal fastening belt for securing patients to a bed, of the type comprising a first strap (1), joined to a fastening band (2) that is adapted to be secured to the bed, with a first section of eyelets (20) arranged successively, and separated a certain distance from each other, and a second strap (3), joined to an abdominal belt (4) with two ends (15, 16) which when they are joined together wrap around and protect the patient's body, with fastening band (2) and abdominal belt (4) also being joined together, **characterized in that** first strap (1) comprises a second section (5) of eyelets, double and unfolded, with at least three eyelets (9, 10, 12) near fastening band (2), with the distance between the first (9) and the second (10) eyelet being different to the distance between the second eyelet (10) and the third one (12).

2. Belt, according to claim 1, **characterized in that** the three eyelets (9, 10, 12) of second section (5) are separated a certain distance according to the distance between the eyelets in first section (20).

3. Belt, according to claim 1, **characterized in that** it has a fourth eyelet (11), arranged on fastening band (2).

4. Belt, according to claim 1, **characterized in that** on abdominal belt (4) internal attachment means (19) have been arranged that secure ends (15,16) of abdominal belt (4) along the perimeter.

5. Belt, according to claim 1, **characterized in that** ends (15,16) of abdominal belt (4) include eyelets (17,18) with identification means (13,14).

## Patentansprüche

1. Unterleib-Befestigungsgurt zum Befestigen von Patienten an einem Bett, bestehend aus einem ersten Streifen (1), der mit einem Halteband (2) verbunden ist, der zur Befestigung am Bett angepaßt ist, mit einem ersten Abschnitt aus nacheinander in einem bestimmten Abstand voneinander angeordneten Ösen (20), und einem zweiten Streifen (3), der mit einem Unterleibgurt (4) mit zwei Enden (15, 16) verbunden ist, die nach ihrer Verbindung um den Körper des Patienten liegen und diesen schützen, wobei das Halteband (2) und der Unterleibgurt (4) auch miteinander verbunden werden, **dadurch gekennzeichnet, daß** der erste Streifen (1) einen zweiten Abschnitt (5) aus doppelten und nicht gefalteten Ösen aufweist, wobei mindestens drei Ösen (9, 10, 12) in der Nähe des Haltebands (2) sind, und sich der Abstand zwischen der ersten (9) und der zweiten Öse (10) vom Abstand zwischen der zweiten Öse (10) und der dritten Öse (12) unterscheidet.

2. Gurt gemäß Anspruch 1, **dadurch gekennzeichnet, daß** die drei Ösen (9, 10, 12) des zweiten Abschnitts (5) einen bestimmten Abstand gemäß dem Abstand zwischen den Ösen im ersten Abschnitt (20) aufweisen.

3. Gurt gemäß Anspruch 1, **dadurch gekennzeichnet, daß** er eine vierte Öse (11) am Halteband (2) aufweist.

4. Gurt gemäß Anspruch 1, **dadurch gekennzeichnet, daß** am Unterleibgurt (4) interne Befestigungsmittel (19) angeordnet wurden, die die Enden (15, 16) des Unterleibgurts (4) um den Umfang sichern.

5. Gurt gemäß Anspruch 1, **dadurch gekennzeichnet, daß** die Enden (15, 16) des Unterleibgurts (4) Ösen (17, 18) mit Kennzeichnungsmitteln (13, 14) aufweisen.

## Revendications

1. Ceinture d'attache abdominale pour le maintien des patients sur le lit, du type comprenant une première courroie (1), unie à une large sangle d'attache (2), qui est adaptée pour être fixée au lit, avec une première section d'oeillets (20) disposés successivement et séparés d'une certaine distance l'un de l'autre, et une deuxième courroie (3), unie à une ceinture abdominale (4) avec deux extrémités (15, 16) et qui, quand elles sont unies ensemble, enveloppent et protègent le corps du patient, la large sangle d'attache (2) et la ceinture abdominale (4) étant également unies ensemble, **caractérisée en ce que** cette première courroie (1) comprend une deuxième section (5) d'oeillets, double et dépliée, avec au moins trois oeillets (9, 10, 12) près de la sangle d'attache (2), la distance entre le premier (9) et le deuxième (10) oeillet étant différente de la distance entre le deuxième (10) et le troisième (12) oeillet.

2. Ceinture conformément à la revendication 1, **caractérisée en ce que** les trois oeillets (9, 10, 12) de la deuxième section (5) sont séparés d'une certaine distance selon la distance entre les oeillets de la première section (20).

3. Ceinture conformément à la revendication 1, **caractérisée en ce qu'**il y a un quatrième oeillet (11) situé sur la large sangle d'attache (2).

4. Ceinture conformément à la revendication 1, **caractérisée en ce que**, sur la ceinture abdominale (4), un système d'attache interne (19) a été conçu qui assure les extrémités (15, 16) de la ceinture abdominale (4) sur tout le périmètre.

5. Ceinture conformément à la revendication 1, **caractérisée en ce que** les extrémités (15, 16) de la ceinture abdominale (4) comprend des oeillets (17, 18) avec un système d'identification (13, 14).
